# EUROPEAN PATENT SPECIFICATION

(11) **EP 4 205 709 B1**
(45) Date of publication and mention of the grant of the patent: **19.06.2024**
(21) Application number: 20950630.2
(22) Date of filing: 26.08.2020
(51) Int. Cl.: A61F 7/02, A61F 7/00, A61H 39/00, A61N 1/04, A61N 1/08, A61N 1/28, A61N 1/32, A61N 1/36

(54) **PATCH HAVING INTELLIGENT DETERMINATION SYSTEM AND USED FOR THERMAL SIGNAL TRANSMISSION**
PATCH MIT INTELLIGENTEM BESTIMMUNGSSYSTEM ZUR THERMISCHEN SIGNALÜBERTRAGUNG
PATCH DOTÉ D'UN SYSTÈME DE DÉTERMINATION INTELLIGENT ET UTILISÉ POUR LA TRANSMISSION DE SIGNAUX THERMIQUES

(43) Date of publication of application: 05.07.2023
(73) Proprietor: Chang, Hui-Ling, New Taipei, Taiwan 234 (TW)
(72) Inventor: LAI, Chien-Hung, New Taipei City 242 (TW); WU, Chang-Sheng, New Taipei City 236 (TW); WANG, Jun-Sheng, New Taipei City 221 (TW); FU, Ke-Chi, Taichung City 408 (TW); CHANG, Chung-Chuan, Kinmen County 890 (TW)
(74) Representative: 2K Patentanwälte Blasberg Kewitz & Reichel
(86) International application number: PCT/CN2020/111238
(87) International publication number: WO 2022/040957

(56) References cited:
- CN-A- 106 994 064
- CN-A- 107 860 483
- CN-A- 109 041 294
- CN-U- 205 126 846
- CN-U- 210 932 172
- CN-U- 210 932 172
- DE-U1-202015 102 429
- US-A1- 2016 015 962

## Description

### BACKGROUND OF THE DISCLOSURE

### Technical Field

The present disclosure relates to an electrode plate which is used for contacting a body and can receive or transmit electrophysiological signals, and especially relates to a thermal signal transmission patch with an intelligent determination system and used for electrotherapy apparatus or measuring physiological signals.

### Description of Related Art

It is known that currently the common sport injuries, degeneration or other problems, with the use of 3C electronic products leading to many bad postures, result in physical pain, such as soreness, pain, numbness and other symptoms due to prolonged bowing and hunchback causing muscle imbalance. Patients will firstly use patches, anti-inflammatory pain relievers or massages to relieve pain once these symptoms occur. However, these methods may not completely cure these symptoms, so patients will seek professional treatment from a physical therapist, or under the evaluation of the physical therapist, patients can purchase physical therapy instruments or apparatuses to use electrotherapy or thermotherapy by themselves.

TW M596618 discloses a patent of a third medicine electrical skin-friendly sheet and electrical connection apparatus, which is an example of the current electrotherapy or thermotherapy apparatus. TW M596618 provides a physical warm moxibustion skin-friendly sheet and related electrical connection apparatus with dual-function physiotherapy of hot compress and electric shock touching human skin; a flexible sheet that has insulating, thermal conductivity properties and can be bent according to the surface of the human body is used; a thermal circuit and a conductive film layer are respectively arranged on two opposite sides of the flexible sheet by plating; the conductive film layer is connected to a superimposed skin-friendly conductive layer; the thermal circuit and the conductive film layer are respectively connected to electricity through a relay terminal, so that the thermal circuit generates thermal temperature; a low frequency pulse current is outputted from the conductive film layer and transmitted to the electricity area which contacts the skin; moreover, the relay terminal is conductive through a conductive button and an opposite button which are dry-typed fastened; the inner circular surfaces of the conductive button and the opposite button are respectively provided with eccentric multi-route electrical conduction structures, which provide a design for connecting power at a multi-angle position; the patch made by the features mentioned above has the function of using both electrotherapy and thermotherapy, but does not have intelligent (namely, without determination function) control for the temperature, and it will heat up endlessly, resulting in burns.

DE 20 2015 102429 U1 discloses a healing apparatus for thermotherapy and electrotherapy, comprising a main body which consists of an inner layer, an outer layer, at least one heating layer and at least two electrode layers. The heating layer is arranged between the inner layer and the outer layer or the two electrode layers are firmly seated on the inner layer. An operating unit which can be attached to the main body or removed therefrom is electrically connected in each case to the heating layer and the two electrode layers.

CN 210 932 172 U discloses an electric-property skin-friendly sheet, which utilizes a flexible mat sheet with insulation and heat-conducting properties and capable of bending according to the human body surface. A hot-work circuit and a conductive film layer are respectively laid on the two opposite surfaces of the flexible mat sheet in a plating manner, the conductive film layer is electrically connected with a superposed skin-friendly conductive layer, and the hot-work circuit and the conductive film layer are respectively electrically connected by a relay terminal.

### SUMMARY OF THE DISCLOSURE

It is an object of the present disclosure to provide a thermal signal transmission patch with an intelligent determination system enabling a fast switching between electrotherapy and thermotherapy and a control of the heating temperature under a specific temperature state during electrotherapy and thermotherapy.

This problem is solved by a thermal signal transmission patch with an intelligent determination system as claimed in claim 1. Further advantageous embodiments are the subject-matter of the dependent claims.

In order to achieve the object mentioned above, the present disclosure provides a thermal signal transmission patch with an intelligent determination system. The thermal signal transmission patch can be electrically connected to an external controller, an external electronic component or an external signal source. The thermal signal transmission patch comprises a substrate and a loop layer. The substrate is a sheet-shaped body. The loop layer is arranged on one side of the substrate. The loop layer at least comprises a first loop layer and a second loop layer. The first loop layer comprises a first contact, a second contact and a third contact. The second loop layer comprises a fourth contact, a fifth contact and a sixth contact. Moreover, an electrical connection mode of the first contact, the second contact, the fourth contact and the fifth contact of the loop layer electrically connected to the external controller or the external electronic component is changed (namely, an electrical connection between the external controller (or the external electronic component), the first contact, the second contact, the fourth contact and the fifth contact is changed; for example, the external controller is configured to change an electrical connection between the first contact, the second contact, the fourth contact and the fifth contact), and the third contact and the sixth contact are electrically connected to the external signal source, so as to achieve (namely, perform) a fast switching between an electrotherapy and a thermotherapy. The first contact is connectable to the second contact, and the fourth contact is connected to the fifth contact, so that a first loop-circuit of the first contact and the second contact is a first pole, and a second loop-circuit of the fourth contact and the fifth contact is a second pole, so as to perform the electrotherapy. The first contact is connectable to the fourth contact, and the second contact is connected to the fifth contact, to form two loop-circuits in parallel for performing the thermotherapy.

In an embodiment of the present disclosure, the first loop layer comprises a first outer line segment and a first inner line segment which is electrically connected to the first outer line segment. The first outer line segment is C-shaped. One end of the first outer line segment is electrically connected to one end of the first inner line segment. The first inner line segment comprises two first straight-line segments and a first C-shaped line segment which is electrically connected between the two first straight-line segments. The other end of the first outer line segment fails to be directly connected to the other end of the first inner line segment; the first contact is formed at the other end of the first outer line segment; the second contact is formed at the other end of the first inner line segment. The third contact is formed where the first outer line segment and the first inner line segment are electrically connected.

In an embodiment of the present disclosure, the second loop layer comprises a second outer line segment and a second inner line segment which is electrically connected to the second outer line segment. The second outer line segment is C-shaped. One end of the second outer line segment is electrically connected to one end of the second inner line segment. The second inner line segment comprises two second straight-line segments and a second C-shaped line segment which is electrically connected between the two second straight-line segments. The other end of the second outer line segment fails to be directly connected to the other end of the second inner line segment; the fourth contact is formed at the other end of the second outer line segment; the fifth contact is formed at the other end of the second inner line segment. The sixth contact is formed where the second outer line segment and the second inner line segment are electrically connected.

In an embodiment of the present disclosure, the first contact and the second contact of the first loop layer and the fourth contact and the fifth contact of the second loop layer are electrically connected to the external controller.

In an embodiment of the present disclosure, the external controller is an active component or a passive component.

In an embodiment of the present disclosure, the active component is a proportional integral derivative controller.

In an embodiment of the present disclosure, the proportional integral derivative controller is a microcontroller, a control chip or a temperature control chip.

In an embodiment of the present disclosure, the passive component is an environmental sensor.

In an embodiment of the present disclosure, the environmental sensor is a thermistor or a relay.

In an embodiment of the present disclosure, the third contact of the first loop layer and the sixth contact of the second loop layer are electrically connected to an electrotherapy-and-hot-compress therapy apparatus.

In an example of the present disclosure, the first loop layer and the second loop layer are connected to form a parallel loop-circuit; the first contact of the first loop layer is connected to the fourth contact of the second loop layer; the second contact of the first loop layer is connected to the fifth contact of the second loop layer; the thermal signal transmission patch further comprises a first thermistor; the second contact and the fifth contact are electrically connected to the first thermistor; the third contact of the first loop layer and the sixth contact of the second loop layer are connected to the external signal source.

In an example of the present disclosure, the thermal signal transmission patch further comprises a third loop layer, a first thermistor and a second thermistor; the third loop layer comprises a first innermost line segment and a second innermost line segment. The first loop layer, the second loop layer and the third loop layer are connected to form a parallel loop-circuit; the first contact of the first loop layer is connected to the fourth contact of the second loop layer; the second contact of the first loop layer is connected to the fifth contact of the second loop layer; the second contact and the fifth contact are electrically connected to the first thermistor; a seventh contact of the first innermost line segment is formed at one end of the first innermost line segment; an eighth contact of the second innermost line segment is formed at one end of the second innermost line segment; the seventh contact is connected to the eighth contact; the seventh contact and the eighth contact are electrically connected to the second thermistor; the other end of the first innermost line segment is electrically connected to the third contact of the first loop layer and the external signal source; the other end of the second innermost line segment is electrically connected to the sixth contact of the second loop layer and the external signal source.

In an embodiment of the present disclosure, the first innermost line segment and the second innermost line segment are two oppositely arranged arcuate shapes.

In an example of the present disclosure, the thermal signal transmission patch further comprises an adhesive layer arranged on the one side of the substrate. The adhesive layer and the loop layer are arranged on the same one side of the substrate.

In an embodiment of the present disclosure, the substrate is an adhesive bandage.

In an embodiment of the present disclosure, the substrate is non-adhesive.

In an embodiment of the present disclosure, the substrate is a bandage.

In an embodiment of the present disclosure, the loop layer is a conductive cloth.

In an embodiment of the present disclosure, the conductive cloth is a silver fiber cloth, nanometer silver wires or a conductive paste.

In an embodiment of the present disclosure, the conductive paste is a thermal-transfer-type conductive gel.

In an embodiment of the present disclosure, the thermal signal transmission patch is a flexible printed circuit board, a flexible line-circuit board, a soft circuit board, a soft line-circuit board, a flexibility line-circuit board or a soft board.

Besides, the present disclosure uses the conductive cloth to manufacture the loop layer of the patch, and achieves a fast switching between electrotherapy and thermotherapy by changing the electrical connection of each contact on the loop layer. With the design of the loop layer, the heating temperature may be controlled under a specific temperature state during electrotherapy and thermotherapy.

Besides, another object of the present disclosure is that the patch made by this method can reduce product risk, and can control the temperature through a low-cost and simple control system during thermotherapy. The present disclosure has an active system or passive system for switching between electrotherapy and thermotherapy, which improves user experience and reduces use hazards; flexibility/softness is increased; the flexibility/softness of a single layer must be higher than that of multiple layers. The present disclosure can be applied to warped areas or body parts with larger curvature. The present disclosure can be used in a single layer or multiple layers for switching between electrotherapy system and thermotherapy system.

### BRIEF DESCRIPTION OF THE DRAWINGS

Fig. 1 shows a schematic plan view of the patch of the first embodiment of the present disclosure.
Fig. 2 shows an exploded schematic view of Fig. 1.
Fig. 3 shows a schematic block diagram of the circuit for electrically connecting the patch of the first embodiment, the external controller and the electrotherapy-and-hot-compress therapy apparatus of the present disclosure.
Fig. 4 shows a schematic plan view of the patch of an example of the present disclosure.
Fig. 5 shows a schematic plan view of the patch of an example of the present disclosure.

### DETAILED DESCRIPTION

Regarding the technical contents and detailed descriptions of the present disclosure, it is now described with the drawings as follows:

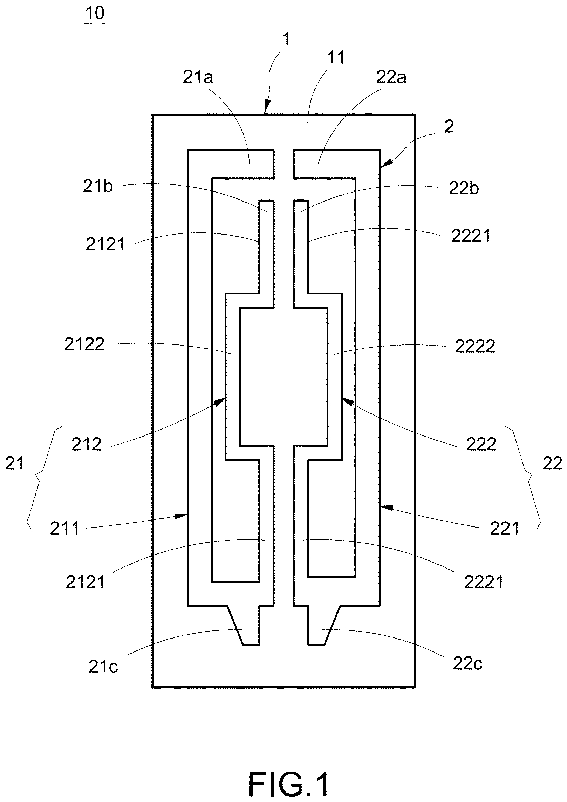

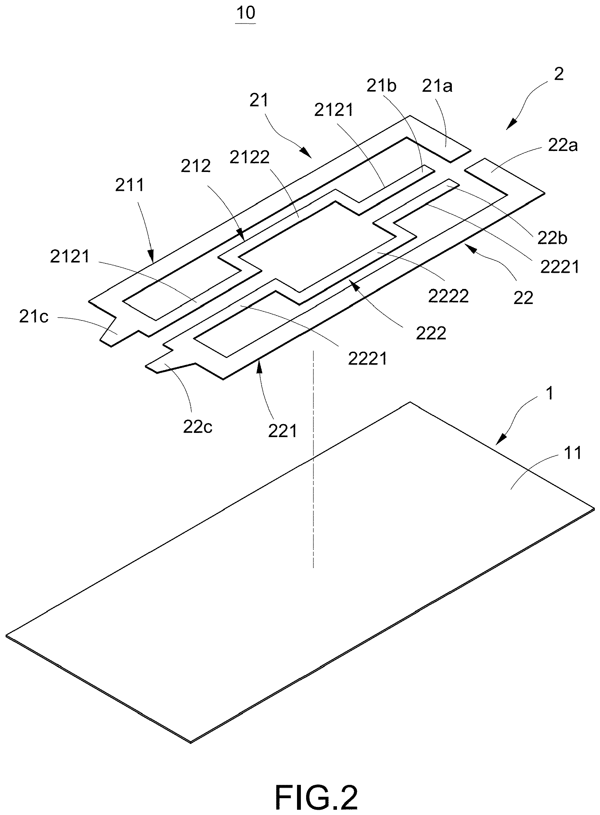

Fig. 1 shows a schematic view of the appearance of the patch of the first embodiment of the present disclosure. Fig. 2 shows an exploded schematic view of the patch of the first embodiment of the present disclosure. As shown in Fig. 1 and Fig. 2, a thermal signal transmission patch (hereinafter referred to as the patch 10) with an intelligent determination system of the present disclosure includes a substrate 1 and a loop layer 2. Moreover, the loop layer 2 includes at least two loops in parallel to form two signal transmission loops. The two signal transmission loops are electrically connected to an external electrotherapy-and-hot-compress therapy apparatus (not shown in the figures) and a controller (not shown in the figures), as the signal transmission (thermal signal transmission) between the external electrotherapy-and-hot-compress therapy apparatus and the controller.

The substrate 1 has no signal transmission function. The patch 10 further includes an adhesive layer 11 arranged on one side of the substrate 1, so that the substrate 1 adheres to the patient's skin surface. In the figures, the substrate 1 is a sheet-shaped body, such as an adhesive bandage material which is the stretchable tape type, while the patch material of the adhesive bandage is a nonwoven cloth with good air permeability, stretchability and flexibility. Besides, the substrate 1 may be a non-adhesive bandage.

The loop layer 2 is arranged on the one side of the substrate 1 with the adhesive layer 11. The loop layer 2 at least includes a first loop layer 21 and a second loop layer 22. The first loop layer 21 includes a first outer line segment 211 and a first inner line segment 212 which is electrically connected to the first outer line segment 211. The first outer line segment 211 is C-shaped. One end of the first outer line segment 211 is electrically connected to one end of the first inner line segment 212. The first inner line segment 212 includes two first straight-line segments 2121 and a first C-shaped line segment 2122 which is electrically connected between the two first straight-line segments 2121. The other end of the first outer line segment 211 fails to be directly connected to the other end of the first inner line segment 212; a first contact 21a is formed at the other end of the first outer line segment 211; a second contact 21b is formed at the other end of the first inner line segment 212. A third contact 21c is formed where the first outer line segment 211 and the first inner line segment 212 are electrically connected. The second loop layer 22 includes a second outer line segment 221 and a second inner line segment 222 which is electrically connected to the second outer line segment 221. The second outer line segment 221 is C-shaped. One end of the second outer line segment 221 is electrically connected to one end of the second inner line segment 222. The second inner line segment 222 includes two second straight-line segments 2221 and a second C-shaped line segment 2222 which is electrically connected between the two second straight-line segments 2221. The other end of the second outer line segment 221 fails to be directly connected to the other end of the second inner line segment 222; a fourth contact 22a is formed at the other end of the second outer line segment 221; a fifth contact 22b is formed at the other end of the second inner line segment 222. A sixth contact 22c is formed where the second outer line segment 221 and the second inner line segment 222 are electrically connected.

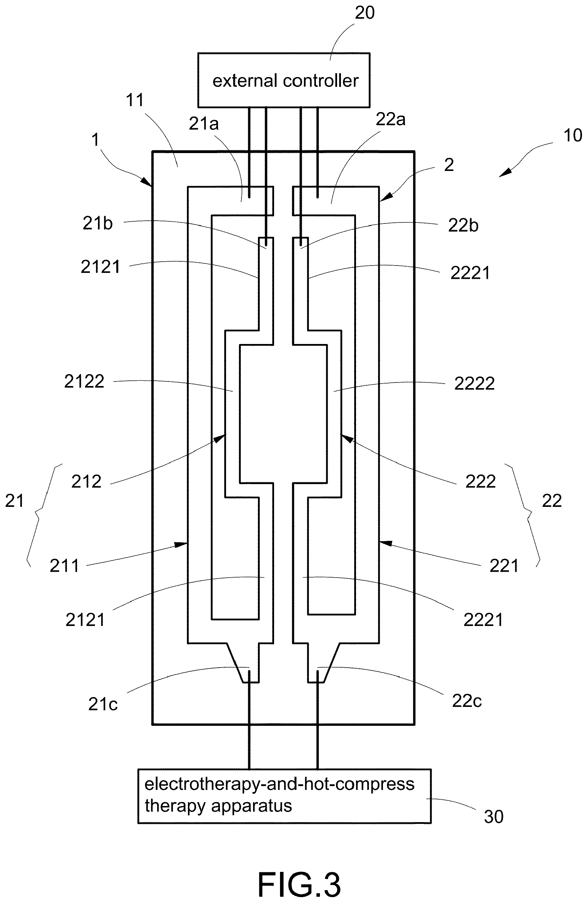

The loop layer 2 mentioned above in the figures is a conductive cloth. The conductive cloth is a carrier with conductive function such as a silver fiber cloth, nanometer silver wires or a conductive paste (such as a thermal-transfer-type conductive gel) printing. The conductive cloth is processed and designed as a specific loop to achieve a single-piece single-layer which can perform thermotherapy treatment and which has the function of simple intelligent temperature control system, wherein the single-layer refers to a layer with a transmission function; other layers without electric signal transmission function may also be laminated on the other end of the main functional layer for modification, but do not affect the main functional layer. The intelligent temperature control refers to a temperature control system that has a determination function and can change due to changes in the external environment; for example, if the temperature of the product reaches 40 degrees, the system determines that the temperature is too high, and the product has other further operations to reduce harm. Fig. 3 shows a schematic block diagram of the circuit for electrically connecting the patch of the first embodiment, the external controller and the electrotherapy-and-hot-compress therapy apparatus of the present disclosure. As shown in Fig. 3, when this embodiment is used, the first contact 21a and the second contact 21b of the first loop layer 21 of the loop layer 2, and the fourth contact 22a and the fifth contact 22b of the second loop layer 22 of the loop layer 2, are electrically connected to the external controller 20, which is an active component or a passive component. The active component is a proportional integral derivative (PID) controller; the proportional integral derivative controller is a microcontroller, a control chip or a temperature control chip. The passive component is an environmental sensor, such as a thermistor or a relay. The third contact 21c of the first loop layer 21 and the sixth contact 22c of the second loop layer 22 are electrically connected to an electrotherapy-and-hot-compress therapy apparatus 30.

In terms of electrotherapy, there are two independent circuits/loops for circuit requirements; for example, the first contact 21a is connected to the second contact 21b, and the fourth contact 22a is connected to the fifth contact 22b, so as to perform the electrotherapy; at this time, a first loop-circuit of the first contact 21a and the second contact 21b is a first pole, and a second loop-circuit of the fourth contact 22a and the fifth contact 22b is a second pole; the electric signal output voltage for electrotherapy is about 40V∼100V pulses, and the output current is 80mA.

In terms of thermotherapy, a parallel loop-circuit is required for circuit requirements; for example, the first contact 21a is connected to the fourth contact 22a, and the second contact 21b is connected to the fifth contact 22b, to form two loop-circuits in parallel; the electric signal output for thermotherapy is the constant voltage (commonly 5V/12V/24V, etc.), the constant current (0.7A/1.0A/1.5A, etc.), or changes in voltage and current according to the heating temperature design, so that the heating temperature can meet the requirement.

Therefore, the electrical connection of the first contact 21a, the second contact 21b, the fourth contact 22a and the fifth contact 22b of the loop layer 2 connected to the external controller 20 is changed (pins jumping/changing), so as to achieve a fast switching between the electrotherapy and the thermotherapy, allowing the user to feel both the electrotherapy and the thermotherapy simultaneously, or switching between the electrotherapy and the thermotherapy. The users are also allowed to adjust the operation mentioned above by themselves.

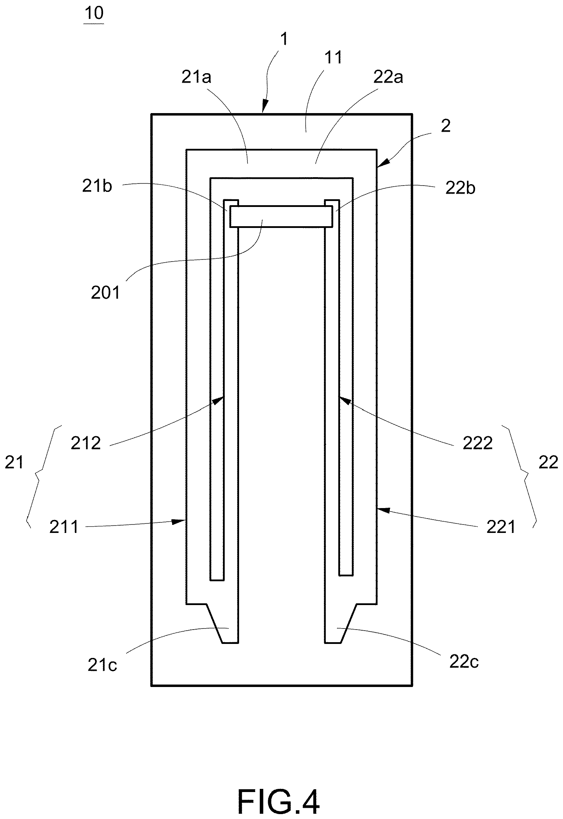

Fig. 4 shows a schematic plan view of the patch of an embodiment not according to the claimed invention.

As shown in Fig. 4, this embodiment is substantially the same as the first embodiment, except that: the first loop layer 21 and the second loop layer 22 are connected to form a parallel loop-circuit; the first contact 21a of the first loop layer 21 is connected to the fourth contact 22a of the second loop layer 22 to form a first coil; the second contact 21b of the first loop layer 21 is connected to the fifth contact 22b of the second loop layer 22; the patch 10 further includes a first thermistor 201; the second contact 21b and the fifth contact 22b are electrically connected to the first thermistor 201 to form a second coil; the third contact 21c of the first loop layer 21 and the sixth contact 22c of the second loop layer 22 form an electrode connection part to be connected to the signal source (the electrotherapy-and-hot-compress therapy apparatus 30).

In these two coils (loop-circuits), the inner loop-circuit resistance is smaller, the outer loop-circuit resistance is larger, while there is the first thermistor 201 in the inner loop-circuit, wherein if the temperature is higher than 40 degrees, the first thermistor 201 is the open circuit; if the temperature is lower than 36 degrees, the thermistor 201 changes back to the short circuit. Because the temperature control range of the first thermistor 201 is not accurate enough and the first thermistor 201 is not sensitive enough, the dual-loop-circuit design is used to make the heating temperature of these two coils not too high and is used to avoid causing damage.

For example, when these two coils are used with a 5V, 1A mobile power supply, the heating efficiency is 5W, the inner loop-circuit resistance is 8.5 ohms, the outer loop-circuit resistance is 12 ohms, and the total resistance including the cable connecting the signal source is about 5 ohms.

The inner loop-circuit resistance is smaller, the line width is smaller, and the heating efficiency (the heat generated per unit area) is stronger, so the inner loop-circuit heats up fast, and the thermistor is triggered by the fast heating through the inner loop-circuit.

When the temperature reaches 40 degrees, the thermistor is cut off; at this time, only the outer loop-circuit continues to generate heat. The outer loop-circuit has a wider line width and a larger resistance; the overall heating efficiency is poor, and the temperature rise is slow, so that the overall system can maintain the temperature, and keep the temperature at 40 degrees (specific temperature).

With the design of the structure of the patch 10 of the present disclosure mentioned above, the patch 10 may be used in medical equipment, sport equipment or rehabilitation aids, such as compression stockings, compression garments, protectors and other highly elastic clothing combined with electrical stimulation (electrotherapy), thermotherapy, or elastic smart clothing (such as with input and output electric signal function or heating function), or thermotherapy products (such as heating eye masks, heating masks, heating blankets and heating clothing).

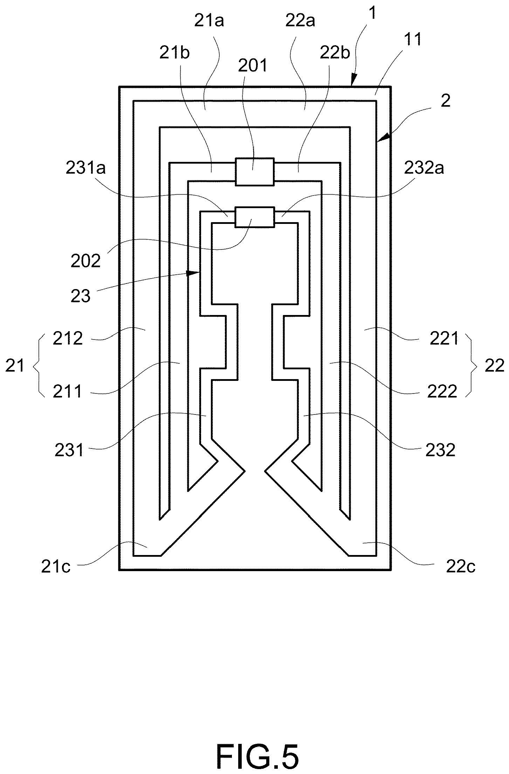

Fig. 5 shows a schematic plan view of the patch of another embodiment not according to the claimed invention.

As shown in Fig. 5, this embodiment is substantially the same as the first embodiment or the second embodiment, except that: the patch 10 further includes a third loop layer 23, a first thermistor 201 and a second thermistor 202; the first loop layer 21, the second loop layer 22 and the third loop layer 23 are connected to form a parallel loop-circuit; the first contact 21a of the first loop layer 21 is connected to the fourth contact 22a of the second loop layer 22 to form the outermost layer coil; the second contact 21b of the first loop layer 21 is connected to the fifth contact 22b of the second loop layer 22; the second contact 21b and the fifth contact 22b are electrically connected to the first thermistor 201 to form the middle layer coil; the third loop layer 23 includes a first innermost line segment 231 and a second innermost line segment 232; the first innermost line segment 231 and the second innermost line segment 232 are two oppositely arranged arcuate shapes; a seventh contact 231a of the first innermost line segment 231 is formed at one end of the first innermost line segment 231; an eighth contact 232a of the second innermost line segment 232 is formed at one end of the second innermost line segment 232; the seventh contact 231a is connected to the eighth contact 232a; the seventh contact 231a and the eighth contact 232a are electrically connected to the second thermistor 202 to form the innermost layer coil; the other end of the first innermost line segment 231 is electrically connected to the third contact 21c of the first loop layer 21 and the external signal source (the electrotherapy-and-hot-compress therapy apparatus 30); the other end of the second innermost line segment 232 is electrically connected to the sixth contact 22c of the second loop layer 22 and the external signal source (the electrotherapy-and-hot-compress therapy apparatus 30).

When this embodiment is used, the resistance value of the outermost layer coil connected as mentioned above is 25Ω (ohms), which is set as a temperature-maintaining coil.

The resistance value of the middle layer coil is 16.67Ω (ohms), which is set as a slow heating coil; with the first thermistor 201, an open circuit is formed when the temperature exceeds 45 degrees.

The resistance value of the innermost layer coil is 10Ω (ohms), which is set as a fast-heating coil; with the second thermistor 202, an open circuit is formed when the temperature exceeds 45 degrees.

Further, the patch 10 of the present disclosure may be manufactured with the flexible printed circuit (FPC) board, which is a special kind of the printed circuit board and is also known as a flexible line-circuit board, a soft circuit board, a soft line-circuit board, a flexibility line-circuit board or a soft board, and so on, so that the patch 10 of the present disclosure has the industrial practicability.

## Claims

1. A thermal signal transmission patch (10), the thermal signal transmission patch (10) being electrically connectable to an external controller (20), an external electronic component or an external signal source, the thermal signal transmission patch (10) comprising:
a substrate (1) being a sheet-shaped body; and
a loop layer (2) arranged on one side of the substrate (1) and at least comprising a first loop layer (21) and a second loop layer (22),
wherein the first loop layer (21) comprises a first contact (21a), a second contact (21b), a third contact (21c), a first outer line segment (211) and a first inner line segment (212) electrically connected to the first outer line segment (211);
wherein the second loop layer (22) comprises a fourth contact (22a), a fifth contact (22b), a sixth contact (22c), a second outer line segment (221) and a second inner line segment (222) electrically connected to the second outer line segment (221), wherein the first outer line segment (211) and the second outer line segment (221) form an outer loop-circuit and the first inner line segment (212) and the second inner line segment (222) form an inner loop-circuit, wherein a resistance of the outer loop-circuit is larger than a resistance of the inner loop-circuit;
wherein, when the third contact (21c) and the sixth contact (22c) are electrically connected to the external signal source, an electrical connection mode of the first contact (21a), the second contact (21b), the fourth contact (22a) and the fifth contact (22b) of the loop layer (2) being electrically connected to the external controller (20) or the external electronic component can be changed, , so as to achieve a fast switching between an electrotherapy and a thermotherapy;
wherein the first contact (21a) is connectable to the second contact (21b) and the fourth contact (22a) is connectable to the fifth contact (22b), so that a first loop-circuit of the first contact (21a) and the second contact (21b) is a first pole, and a second loop-circuit of the fourth contact (22a) and the fifth contact (22b) is a second pole, so as to perform the electrotherapy;
wherein the first contact (21a) is connectable to the fourth contact (22a) and the second contact (21b) is connectable to the fifth contact (22b), to form two loop-circuits in parallel for performing the thermotherapy.

2. The thermal signal transmission patch (10) of claim 1, wherein the first outer line segment (211) is C-shaped; one end of the first outer line segment (211) is electrically connected to one end of the first inner line segment (212); the first inner line segment (212) comprises two first straight line segments (2121) and a first C-shaped line segment (2122) electrically connected between the two first straight line segments (2121); the other end of the first outer line segment (211) fails to be directly connected to the other end of the first inner line segment (212); the first contact (21a) is formed at the other end of the first outer line segment (211); the second contact (21b) is formed at the other end of the first inner line segment (212); the third contact (21c) is formed where the first outer line segment (211) and the first inner line segment (212) are electrically connected.

3. The thermal signal transmission patch (10) of claim 2, wherein the second outer line segment (221) is C-shaped; one end of the second outer line segment (221) is electrically connected to one end of the second inner line segment (222); the second inner line segment (222) comprises two second straight line segments (2221) and a second C-shaped line segment (2222) electrically connected between the two second straight line segments (2221); the other end of the second outer line segment (221) fails to be directly connected to the other end of the second inner line segment (222); the fourth contact (22a) is formed at the other end of the second outer line segment (221); the fifth contact (22b) is formed at the other end of the second inner line segment (222); the sixth contact (22c) is formed where the second outer line segment (221) and the second inner line segment (222) are electrically connected.

4. The thermal signal transmission patch (10) of claim 1, wherein the first contact (21a) and the second contact (21b) of the first loop layer (21) and the fourth contact (22a) and the fifth contact (22b) of the second loop layer (22) are electrically connectable to the external controller (20).

5. The thermal signal transmission patch (10) of claim 1, wherein the external controller (20) is a proportional integral derivative controller.

6. The thermal signal transmission patch (10) of claim 1, wherein the third contact (21c) of the first loop layer (21) and the sixth contact (22c) of the second loop layer (22) are electrically connectable to an electrotherapy-and-hot-compress therapy apparatus (30).

7. The thermal signal transmission patch (10) of claim 1, further comprising an adhesive layer (11) arranged on the one side of the substrate (1), wherein the adhesive layer (11) and the loop layer (2) are arranged on the same one side of the substrate (1).

8. The thermal signal transmission patch (10) of claim 7, wherein the substrate (1) is an adhesive bandage.

9. The thermal signal transmission patch (10) of claim 1, wherein the loop layer (2) is a conductive cloth.

10. The thermal signal transmission patch (10) of claim 1, wherein the thermal signal transmission patch (10) is a flexible printed circuit board, a flexible line-circuit board, a soft circuit board, a soft line-circuit board, a flexibility line-circuit board or a soft board.

## Patentansprüche

1. Wärmesignalübertragungs-Pflaster (10), wobei das Wärmesignalübertragungs-Pflaster (10) mit einer externen Steuerung (20), einem externen elektronischen Bauelement oder einer externen Signalquelle elektrisch verbindbar ist, wobei das Wärmesignalübertragungs-Pflaster (10) umfasst:
ein Substrat (1), das ein blattförmiger Körper ist; und
eine Leitungsschleifenschicht (2), die auf einer Seite des Substrats (1) angeordnet ist und mindestens eine erste Leitungsschleifenschicht (21) und eine zweite Leitungsschleifenschicht (22) umfasst,
wobei die erste Leitungsschleifenschicht (21) einen ersten Kontakt (21a), einen zweiten Kontakt (21b), einen dritten Kontakt (21c), ein erstes äußeres Leitungssegment (211) und ein erstes inneres Leitungssegment (212), das elektrisch mit dem ersten äußeren Leitungssegment (211) verbunden ist, umfasst
wobei die zweite Leitungsschleifenschicht (22) einen vierten Kontakt (22a), einen fünften Kontakt (22b), einen sechsten Kontakt (22c), ein zweites äußeres Leitungssegment (221) und ein zweites inneres Leitungssegment (222) umfasst, das elektrisch mit dem zweiten äußeren Leitungssegment (221) verbunden ist, wobei das erste äußere Leitungssegment (211) und das zweite äußere Leitungssegment (221) einen äußeren Leitungsschleifenstromkreis bilden und das erste innere Leitungssegment (212) und das zweite innere Leitungssegment (222) einen inneren Leitungsschleifenstromkreis bilden, wobei ein Widerstand des äußeren Leitungsschleifenstromkreises größer ist als ein Widerstand des inneren Leitungsschleifenstromkreises;
wobei, wenn der dritte Kontakt (21c) und der sechste Kontakt (22c) elektrisch mit der externen Signalquelle verbunden sind, ein elektrischer Verbindungsmodus des ersten Kontakts (21a), des zweiten Kontakts (21b), des vierten Kontakts (22a) und des fünften Kontakts (22b) der Leitungsschleifenschicht (2), die elektrisch mit der externen Steuerung (20) oder der externen elektronischen Bauelement verbunden sind, geändert werden kann, um ein schnelles Umschalten zwischen einer Elektrotherapie und einer Thermotherapie zu erreichen;
wobei der erste Kontakt (21a) mit dem zweiten Kontakt (21b) verbindbar ist und der vierte Kontakt (22a) mit dem fünften Kontakt (22b) verbindbar ist, so dass ein erster Leitungsschleifenstromkreis des ersten Kontakts (21a) und des zweiten Kontakts (21b) einen ersten Pol einen und ein zweiter Leitungsschleifenstromkreis des vierten Kontakts (22a) und des fünften Kontakts (22b) einen zweiten Pol bilden, um die Elektrotherapie durchzuführen;
wobei der erste Kontakt (21a) mit dem vierten Kontakt (22a) verbindbar ist und der zweite Kontakt (21b) mit dem fünften Kontakt (22b) verbindbar ist, um zwei parallele Leitungsschleifenschaltungen zur Durchführung der Thermotherapie zu bilden.

2. Wärmesignalübertragungs-Pflaster (10) nach Anspruch 1, wobei das erste äußere Leitungssegment (211) C-förmig ist; ein Ende des ersten äußeren Leitungssegments (211) elektrisch mit einem Ende des ersten inneren Leitungssegments (212) verbunden ist; das erste innere Leitungssegment (212) zwei erste gerade Leitungssegmente (2121) und ein erstes C-förmiges Leitungssegment (2122) umfasst, das elektrisch zwischen den beiden ersten geraden Leitungssegmenten (2121) verbunden ist; das andere Ende des ersten äußeren Leitungssegments (211) nicht unmittelbar mit dem anderen Ende des ersten inneren Leitungssegments (212) verbunden ist; der erste Kontakt (21a) an dem anderen Ende des ersten äußeren Leitungssegments (211) ausgebildet ist; der zweite Kontakt (21b) an dem anderen Ende des ersten inneren Leitungssegments (212) ausgebildet ist; der dritte Kontakt (21c) dort ausgebildet ist, wo das erste äußere Leitungssegment (211) und das erste innere Leitungssegment (212) elektrisch verbunden sind.

3. Wärmesignalübertragungs-Pflaster (10) nach Anspruch 2, wobei das zweite äußere Leitungssegment (221) C-förmig ist; ein Ende des zweiten äußeren Leitungssegments (221) elektrisch mit einem Ende des zweiten inneren Leitungssegments (222) verbunden ist; das zweite innere Leitungssegment (222) zwei zweite gerade Leitungssegmente (2221) und ein zweites C-förmiges Leitungssegment (2222) umfasst, das elektrisch zwischen den beiden zweiten geraden Leitungssegmenten (2221) verbunden ist; das andere Ende des zweiten äußeren Leitungssegments (221) nicht unmittelbar mit dem anderen Ende des zweiten inneren Leitungssegments (222) verbunden ist; der vierte Kontakt (22a) an dem anderen Ende des zweiten äußeren Leitungssegments (221) ausgebildet ist; der fünfte Kontakt (22b) an dem anderen Ende des zweiten inneren Leitungssegments (222) ausgebildet ist; der sechste Kontakt (22c) dort ausgebildet ist, wo das zweite äußere Leitungssegment (221) und das zweite innere Leitungssegment (222) elektrisch miteinander verbunden sind.

4. Wärmesignalübertragungs-Pflaster (10) nach Anspruch 1, wobei der erste Kontakt (21a) und der zweite Kontakt (21b) der ersten Leitungsschleifenschicht (21) und der vierte Kontakt (22a) und der fünfte Kontakt (22b) der zweiten Leitungsschleifenschicht (22) elektrisch mit der externen Steuerung (20) verbindbar sind.

5. Wärmesignalübertragungs-Pflaster (10) nach Anspruch 1, wobei die externe Steuerung (20) ein Proportional-Integral-Differenzial-Regler ist.

6. Wärmesignalübertragungs-Pflaster (10) nach Anspruch 1, wobei der dritte Kontakt (21c) der ersten Leitungsschleifenschicht (21) und der sechste Kontakt (22c) der zweiten Leitungsschleifenschicht (22) elektrisch mit einem Elektrotherapie- und Wärmekompressions-Therapiegerät (30) verbindbar sind.

7. Wärmesignalübertragungs-Pflaster (10) nach Anspruch 1, das ferner eine Klebstoffschicht (11) umfasst, die auf der einen Seite des Substrats (1) angeordnet ist, wobei die Klebstoffschicht (11) und die Leitungsschleifenschicht (2) auf derselben einen Seite des Substrats (1) angeordnet sind.

8. Wärmesignalübertragungs-Pflaster (10) nach Anspruch 7, wobei das Substrat (1) eine Klebebandage ist.

9. Wärmesignalübertragungs-Pflaster (10) nach Anspruch 1, wobei die Leitungsschleifenschicht (2) ein leitfähiger Stoff ist.

10. Wärmesignalübertragungs-Pflaster (10) nach Anspruch 1, wobei das Wärmesignalübertragungs-Pflaster (10) eine flexible Leiterplatte, eine flexible Leitungsplatine, eine weiche Leiterplatte, eine weiche Leitungsplatine, eine flexible Leitungsplatine oder eine weiche Platte ist.

## Revendications

1. Un patch pour la transmission de signaux thermiques (10), le patch pour la transmission de signaux thermiques (10) pouvant être connecté électriquement à un contrôleur externe (20), à un élément électronique externe ou à une source de signal externe, le pour la transmission de signaux thermiques (10) comprenant :
un substrat (1) en forme de feuille ; et
une couche de circuits (2) disposée sur un côté du substrat (1) et comprenant au moins une première couche de circuits (21) et une seconde couche de circuits (22),
dans laquelle la première couche de circuits (21) comprend un premier contact (21a), un deuxième contact (21b), un troisième contact (21c), un premier segment de ligne externe (211) et un premier segment de ligne interne (212) connectés électriquement au premier segment de ligne externe (211) ;
dans laquelle la deuxième couche de circuits (22) comprend un quatrième contact (22a), un cinquième contact (22b), un sixième contact (22c), un deuxième segment de ligne externe (221) et un deuxième segment de ligne interne (222) connectés électriquement au deuxième segment de ligne externe (221), dans lequel le premier segment de ligne externe (211) et le deuxième segment de ligne externe (221) forment un circuit en boucle externe et le premier segment de ligne interne (212) et le deuxième segment de ligne interne (222) forment un circuit en boucle interne, dans lequel une résistance du circuit en boucle externe est plus grande qu'une résistance du circuit en boucle interne ;
dans lequel, lorsque le troisième contact (21c) et le sixième contact (22c) sont connectés électriquement à la source de signal externe, un mode de connexion électrique du premier contact (21a), du deuxième contact (21b), du quatrième contact (22a) et du cinquième contact (22b) de la couche de circuits (2) étant connecté électriquement au contrôleur externe (20) ou au élément électronique externe peut être modifié, de manière à réaliser une commutation rapide entre une électrothérapie et une thermothérapie ;
dans lequel le premier contact (21a) peut être connecté au deuxième contact (21b) et le quatrième contact (22a) peut être connecté au cinquième contact (22b), de sorte qu'un premier circuit en boucle du premier contact (21a) et du deuxième contact (21b) constitue un premier pôle, et qu'un deuxième circuit en boucle du quatrième contact (22a) et du cinquième contact (22b) constitue un deuxième pôle, afin d'effectuer l'électrothérapie ;
le premier contact (21a) peut être connecté au quatrième contact (22a) et le deuxième contact (21b) peut être connecté au cinquième contact (22b), afin de former deux circuits en boucle en parallèle pour effectuer la thermothérapie.

2. Le patch pour la transmission de signaux thermiques (10) selon la revendication 1, dans lequel le premier segment de ligne externe (211) est en forme de C ; une extrémité du premier segment de ligne externe (211) est connectée électriquement à une extrémité du premier segment de ligne interne (212) ; le premier segment de ligne interne (212) comprend deux premiers segments de ligne droite (2121) et un premier segment de ligne en forme de C (2122) connecté électriquement entre les deux premiers segments de ligne droite (2121) ; l'autre extrémité du premier segment de ligne extérieur (211) n'est pas directement connectée à l'autre extrémité du premier segment de ligne intérieur (212) ; le premier contact (21a) est formé à l'autre extrémité du premier segment de ligne extérieur (211) ; le deuxième contact (21b) est formé à l'autre extrémité du premier segment de ligne intérieur (212) ; le troisième contact (21c) est formé à l'endroit où le premier segment de ligne extérieur (211) et le premier segment de ligne intérieur (212) sont électriquement connectés.

3. Le patch pour la transmission de signaux thermiques (10) selon la revendication 2, dans lequel le deuxième segment de ligne externe (221) est en forme de C ; une extrémité du deuxième segment de ligne externe (221) est connectée électriquement à une extrémité du deuxième segment de ligne interne (222) ; le deuxième segment de ligne interne (222) comprend deux deuxièmes segments de ligne droite (2221) et un deuxième segment de ligne en forme de C (2222) connecté électriquement entre les deux deux deuxièmes segments de ligne droite (2221) ; l'autre extrémité du deuxième segment de ligne extérieur (221) n'est pas directement connectée à l'autre extrémité du deuxième segment de ligne intérieur (222) ; le quatrième contact (22a) est formé à l'autre extrémité du deuxième segment de ligne extérieur (221) ; le cinquième contact (22b) est formé à l'autre extrémité du deuxième segment de ligne intérieur (222) ; le sixième contact (22c) est formé à l'endroit où le deuxième segment de ligne extérieur (221) et le deuxième segment de ligne intérieur (222) sont électriquement connectés.

4. Le patch pour la transmission de signaux thermiques (10) selon la revendication 1, dans lequel le premier contact (21a) et le deuxième contact (21b) de la première couche de circuits (21) et le quatrième contact (22a) et le cinquième contact (22b) de la deuxième couche de circuits (22) sont électriquement connectables au contrôleur externe (20).

5. Le patch pour la transmission de signaux thermiques (10) selon la revendication 1, dans lequel le contrôleur externe (20) est un contrôleur dérivé proportionnel intégral.

6. Le patch pour la transmission de signaux thermiques (10) selon la revendication 1, dans lequel le troisième contact (21c) de la première couche de circuits (21) et le sixième contact (22c) de la deuxième couche de circuits (22) sont électriquement connectables à un appareil d'électrothérapie et de thérapie par chaud-compression (30).

7. Le patch pour la transmission de signaux thermiques (10) selon la revendication 1, comprenant en outre une couche adhésive (11) disposée sur la première face du substrat (1), la couche adhésive (11) et la couche à boucles (2) étant disposées sur la même première face du substrat (1).

8. Le patch pour la transmission de signaux thermiques (10) selon la revendication 7, dans lequel le substrat (1) est un bandage adhésif.

9. Le patch pour la transmission de signaux thermiques (10) selon la revendication 1, dans lequel la couche de circuits (2) est un tissu conducteur.

10. Le patch pour la transmission de signaux thermiques (10) selon la revendication 1, dans lequel le patch de transmission de signal thermique (10) est une carte de circuit imprimé flexible, une carte de circuit de ligne flexible, une carte de circuit souple, une carte de circuit de ligne souple, une carte de circuit de ligne de flexibilité ou une carte souple.
